# EUROPEAN PATENT APPLICATION

(11) **EP 1 749 833 A1**
(43) Date of publication of application: **07.02.2007**
(21) Application number: 05254884.9
(22) Date of filing: 05.08.2005
(51) Int. Cl.: C07K 14/165, C07K 19/00, A61K 38/16, C07H 21/00

(54) **Super-antigens derived from the SARS coronavirus E2 spike protein**

(71) Applicant: Healthbanks Biotech Co., Ltd., Taipei City (TW)
(72) Inventor: Chang, Hsiu-Kang, Da-an District, Taipei City 106 (TW); Liao, Chao-We, Jhunan Township Miaoli County 350 (TW); Cheng, Wen-Fang, Taipei City 110 (TW)
(74) Representative: Tranter, Andrew David

(57) **Abstract**

The present invention relates to a super-antigen fusion protein, comprising: a peptide fragment derived from SARS E2 spike protein; and a translocating peptide fragment for transporting a protein into a cell and translocating the protein in cytosol; wherein, the SARS E2 spike protein fragments locate between the amino acid position 692 and 1050 of the viral protein. The present invention further relates to DNA sequences encoding the SARS E2 spike protein fragments and fusion protein thereof; wherein the DNA sequences are able to express the proteins in an *E. Coli* expression system. The preferred translocating peptide is obtained from *Pseudomonas* exotoxin.

SEQUENCE LISTING

<110>  HealthBanks

<120>  A superantigen fusion protein and the use thereof

<130>  P6160/0613

<160>  8

<170>  PatentIn version 3.3

<210>  1
<211>  71
<212>  PRT
<213>  Coronavirus E2 spikeprotein

<300>
<308>  NP_828851
<309>  2004-04-09
<313>  (1)..(71)

<400>  1

Asn Thr Ile Ala Ile Pro Thr Asn Phe Ser Ile Ser Ile Thr Thr Glu
1               5                   10                  15

Val Met Pro Val Ser Met Ala Lys Thr Ser Val Asp Cys Asn Met Tyr
            20                  25                  30

Ile Cys Gly Asp Ser Thr Glu Cys Ala Asn Leu Leu Leu Gln Tyr Gly
        35                  40                  45

Ser Phe Cys Thr Gln Leu Asn Arg Ala Leu Ser Gly Ile Ala Ala Glu
    50                  55                  60

Gln Asp Arg Asn Thr Arg Glu
65                  70

<210>  2
<211>  97
<212>  PRT
<213>  Coronavirus E2 spikeprotein

<300>
<308>  NP_828851
<309>  2004-04-09
<313>  (1)..(97)

<400>  2

Val Phe Ala Gln Val Lys Gln Met Tyr Lys Thr Pro Thr Leu Lys Tyr
1               5                   10                  15

Phe Gly Gly Phe Asn Phe Ser Gln Ile Leu Pro Asp Pro Leu Lys Pro
            20                  25                  30

Thr Lys Arg Ser Phe Ile Glu Asp Leu Leu Phe Asn Lys Val Thr Leu
        35                  40                  45

Ala Asp Ala Gly Phe Met Lys Gln Tyr Gly Glu Cys Leu Gly Asp Ile
    50                  55                  60

Asn Ala Arg Asp Leu Ile Cys Ala Gln Lys Phe Asn Gly Leu Thr Val
65                  70                  75                  80

Leu Pro Pro Leu Leu Thr Asp Asp Met Ile Ala Ala Tyr Thr Ala Ala
                85                  90                  95

Leu

<210>  3
<211>  90
<212>  PRT
<213>  Coronavirus E2 spikeprotein

<300>
<308>  NP_828851
<309>  2004-04-09
<313>  (1)..(90)

<400>  3

Val Ser Gly Thr Ala Thr Ala Gly Trp Thr Phe Gly Ala Gly Ala Ala
1               5                   10                  15

Leu Gln Ile Pro Phe Ala Met Gln Met Ala Tyr Arg Phe Asn Gly Ile
            20                  25                  30

Gly Val Thr Gln Asn Val Leu Tyr Glu Asn Gln Lys Gln Ile Ala Asn
        35                  40                  45

Gln Phe Asn Lys Ala Ile Ser Gln Ile Gln Glu Ser Leu Thr Thr Thr
    50                  55                  60

Ser Thr Ala Leu Gly Lys Leu Gln Asp Val Val Asn Gln Asn Ala Gln
65                  70                  75                  80

Ala Leu Asn Thr Leu Val Lys Gln Leu Ser
                85                  90

<210>  4
<211>  101
<212>  PRT
<213>  Coronavirus E2 spikeprotein

<300>
<308>  NP_828851
<309>  2004-04-09
<313>  (1)..(101)

<400>  4

Ser Asn Phe Gly Ala Ile Ser Ser Val Leu Asn Asp Ile Leu Ser Arg
1               5                   10                  15

Leu Asp Lys Val Glu Ala Glu Val Gln Ile Asp Arg Leu Ile Thr Gly
            20                  25                  30

Arg Leu Gln Ser Leu Gln Thr Tyr Val Thr Gln Gln Leu Ile Arg Ala
        35                  40                  45

Ala Glu Ile Arg Ala Ser Ala Asn Leu Ala Ala Thr Lys Met Ser Glu
    50                  55                  60

Cys Val Leu Gly Gln Ser Lys Arg Val Asp Phe Cys Gly Lys Gly Tyr
65                  70                  75                  80

His Leu Met Ser Phe Pro Gln Ala Ala Pro His Gly Val Val Phe Leu
                85                  90                  95

His Val Thr Tyr Val
            100

<210>  5
<211>  219
<212>  DNA
<213>  Artificial

<220>
<223>  DNA encoding partial SARS E2 spike protein expressed in E.coli
       system.

<400>  5
aacaccatcg ctatcccgac caacttctcc atctccatca ccaccgaagt tatgccggtt      60

tccatggcta aaacctccgt tgactgcaac atgtacatct gcggtgactc caccgaatgc     120

gctaacctgc tgctgcagta cggttccttc tgcacccagc tgaaccgtgc tctgtccggt     180

atcgctgctg aacaggaccg taacacccgt gaataatag                            219

<210>  6
<211>  297
<212>  DNA
<213>  Artificial

<220>
<223>  DNA encoding partial SARS E2 spike protein expressed in E.coli
       system.

<400>  6
gttttcgctc aggttaaaca gatgtacaaa accccgaccc tgaaatactt cggtggtttc      60

aacttctccc agatcctgcc ggacccgctg aaaccgacca aacgttcctt catcgaagac     120

ctgctgttca acaaagttac cctggctgac gctggtttca tgaaacagta cggtgaatgc     180

ctgggtgaca tcaacgctcg tgacctgatc tgcgctcaga aattcaacgg tctgaccgtt     240

ctgccgccgc tgctgaccga cgacatgatc gctgcttaca ccgctgctct gtaatag        297

<210>  7
<211>  276
<212>  DNA
<213>  Artificial

<220>
<223>  DNA encoding partial SARS E2 spike protein expressed in E.coli
       system

<400>  7
gtttccggta ccgctaccgc tggttggacc ttcggtgctg gtgctgctct gcagatcccg      60

ttcgctatgc agatggctta ccgtttcaac ggtatcggtg ttacccagaa cgttctgtac     120

gaaaaccaga aacagatcgc taaccagttc aacaaagcta tctcccagat ccaggaatcc     180

ctgaccacca cctccaccgc tctgggtaaa ctgcaggacg ttgttaacca gaacgctcag     240

gctctgaaca ccctggttaa acagctgtcc taatag                               276


<210>  8
<211>  309
<212>  DNA
<213>  Artificial

<220>
<223>  DNA encoding partial SARS E2 spike protein expressed in E.coli
       system.

<400>  8
tccaacttcg gtgctatctc ctccgttctg aacgacatcc tgtcccgtct ggacaaagtt      60

gaagctgaag ttcagatcga ccgtctgatc accggtcgtc tgcagtccct gcagacctac     120

gttacccagc agctgatccg tgctgctgaa atccgtgctt ccgctaacct ggctgctacc     180

aaaatgtccg aatgcgttct gggtcagtcc aaacgtgttg acttctgcgg taaaggttac     240

cacctgatgt ccttcccgca ggctgctccg cacggtgttg ttttcctgca cgttacctac     300

gtttaatag                                                             309

## Description

### BACKGROUND OF THE INVENTION

### 1. Field of the Invention

The present invention relates to a super-antigen fusion protein and the use thereof and, more particularly, to a fusion protein which binds to an antigen-presenting cell for inducing antibodies effectively, so as to suppress the virus infection and block the super-antigen response.

### 2. Description of Related Art

A virus is too small to propagate independently, and has to survive in a host cells by way of parasitism. Hence, viruses are classified in a species between organism and non-organism. Coronavirus is a single-strand RNA virus, and the way it enters cells is by binding to a target cell receptor and forming a cyst, then the virus enters host cells through endocytosis. The viral ribonucleic acid (RNA) reverses to DNA via reverse transcription, and further inserts into chromosomes of the host cell. Therefore, the proteins and genetic materials required for virus growth are produced by the host cell. Under the enzyme catalysis, proteins and genetic materials are assembled and released from the cell, thus the host cell is destroyed.

The virus recognizes the receptors on a cell surface, for example, HIV recognizes CD4 and CCR5 antigens on T lymphocyte surface and then enters the cell. However, coronavirus recognizes aminopeptidase-N on lung or kidney cell surfaces, which is the structure of CD 13 receptor, for invasion. Therefore, the main direction in developing biotechnology against viruses is to design a drug to interrupt the interaction between virus and surface antigens, so as to prevent virus invasion. However, besides the structure of the above-mentioned for pathogenesis, some virus has another portion just like a super-antigen to interact with host immune system, which directly binds to a T lymphocyte surface receptor, and then induces interleukin or γ-interferon production heavily from T lymphocyte, resulting in drastically inflammatory response and even triggering the linked T lymphocyte to death (such as programmed cell death apoptosis). Therefore, by elimination of the binding elements of a binding event between super-antigen and cell surface antigen, the viral invasion is suppressed, and the infection symptoms is possibly prevented or alleviated.

Each T lymphocyte membrane exhibits its own T lymphocyte receptor (TCR) in the immune system. There are approximately one million mature T lymphocytes patrolling in the human body. Therefore, TCR is used for monitoring the messages from cells or antigen-presenting cell membranes in the body. An antigen-presenting cell exhibits a Major Histocomparibility Complex (MHC), which is capable of recognizing foreign proteins. MHC and a peptide fragment will bind together to form a complex, and then present on the surface of cell membrane. Thus, the complex formed of MHC and peptide will specifically release a message to TCR, which acts as a mediator for self and non-self recognition. However, from the scientific research, it is found that a certain part of the SARS (severe acute respiratory syndrome) virus envelope spike protein will bind to a T lymphocyte receptor after invasion. Moreover, the message of mis-recognizing the immune cell result in a releasing cytokines immediately without the involvement of MHC molecule through an antigen-presenting cell system. Therefore, T lymphocytes are induced to largely proliferate or produce vast amount of cytokines, which attack vigorously back to cells and result in inflammatory response. Based on the above study, it is sure that the SARS spike protein exhibits the "super-antigen" property. However, the position of amino acid sequence where the super-antigen fragment locates is still under investigation. Recently, it has been speculated that the super-antigen position locates at the SARS spike protein amino acid sequence 680 to 1050, however, the exactitude needs to be further confirmed.

To prevent the autoimmunological response resulting from SARS virus invasion, a vaccine-like protection system can be utilized. The key point of vaccination is to trigger the originally existing antibody of the infected host with the lymph cell that carries memories of invasion for response. The inventor of the present invention found that the strategy of "induction antibody effectively with a fusion protein" can proceed to a "partial immunization of a super-antigen". The strategy is designed to provide a fusion protein with partial super-antigen of the SARS virus to a healthy human, and a higher titer of antibody is anticipated, wherein the antibody is induced from the immune cells which are capable of recognizing SARS virus super-antigen regions. During infection, the antibody will capture the super-antigen region of SARS virus and this results in alleviating super-antigen induction or inflammatory response without over-stimulating T lymphocyte proliferation. The inventor of the present invention also utilizes the prior investigation of "fusion protein transporting system", to proceed the "antibody induction of CD 13 cell receptor binding region". Thus, the induced antibody can suppress SARS virus invasion and the SARS infection is prevented.

For the proceedings of the SARS virus investigation, the first concern is to obtain the SARS virus. However, the virus is transmitted by droplet contact, thus the highly infectious virus must be isolated in an appropriately-equipped laboratory. Even though scientists have already sequenced the wild-type SARS virus genome, an *ex vivo* synthesis of vast amount of viruses is still dependent on a specific host system, which limits the investigation. In the present invention, the inventor translates the amino acid sequence of SARS wild-type virus into specific protein which is published via the Internet. The codons in the amino acid sequence of the SARS virus are modified with translating-effective codons of the ordinary *E. Coli* host system, and the modified sequence is synthesized by sequential PCR. SARS proteins are further expressed effectively by a conventional E. *Coli* host system. The present invention aims to acquire a SARS antigen without adopting a virus gene entity, which is beneficial for SARS virus research.

### SUMMARY OF THE INVENTION

The present invention provides a super-antigen fusion protein and the application method thereof, wherein the super-antigen fusion protein comprises a peptide fragment encoding the SARS E2 spike protein, and a translocating peptide. In order to *ex vivo* obtain the SARS E2 spike protein without infection of the highly infectious virus, the present invention further comprises a nucleic acid sequence encoding super-antigen fusion protein to be expressed as the target protein in the *E. Coli* expression system.

Another object of the present invention is to provide a super-antigen peptide domain which is able to prevent the binding of SARS virus with a T lymphocyte, especially for a peptide domain targeting the E2 spike protein of the SARS virus. Similar to the way of antibody recognition, an E2 spike protein super-antigen peptide domain binds to the position of T lymphocyte where the invasive SARS virus are expected to bind. As a result, immunological responses and allergic reactions are prevented excessive when the invasive SARS virus binds to the T lymphocyte.

Another object of the present invention is to provide a peptide binding domain which prevents the binding of the virus with the T lymphocyte surface receptor CD13, specifically for a peptide targeting the SARS E2 spike protein. Similar to the way of antibody recognition, an E2 spike protein super-antigen peptide domain binds to the position of the T lymphocyte surface receptor CD 13 where the invasive SARS virus is expected to bind. The binding mechanism between a virus and the immune T lymphocyte surface receptor CD 13 is blocked, which prevents SARS virus invasion through the immune T lymphocyte CD 13 binding.

The present invention can be used for detecting SARS virus infection. The induced antibody stimulated by the fusion protein of the present invention prevents the host T lymphocyte from being infected by a virus , and reduces the excessive immunological response and allergic reaction resulting from immune T lymphocyte and SARS virus binding. Therefore, the fusion protein of the present invention is also used for relieving or treating the infection symptoms of the SARS virus.

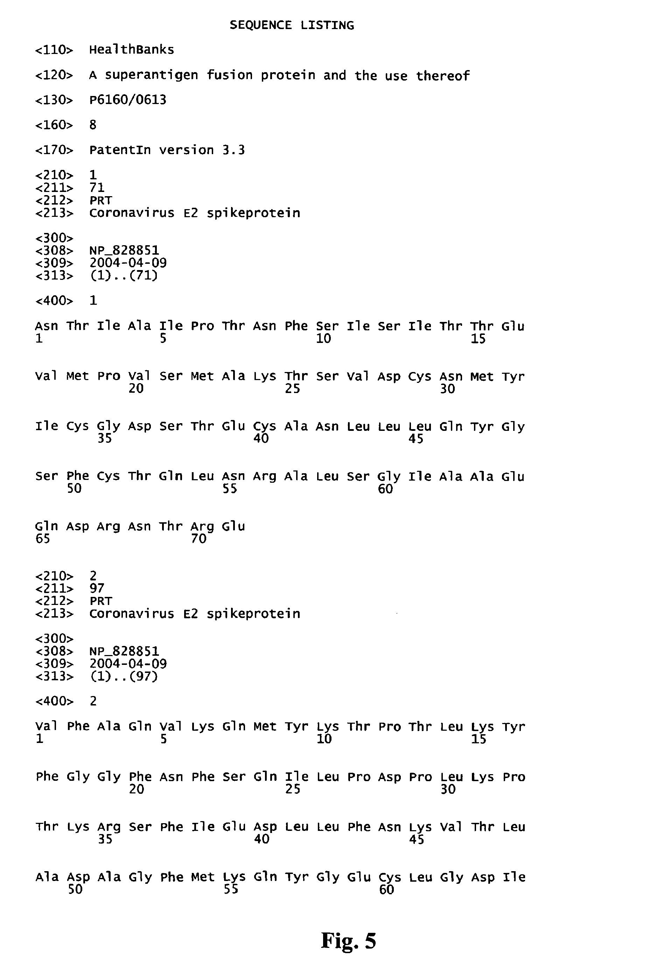

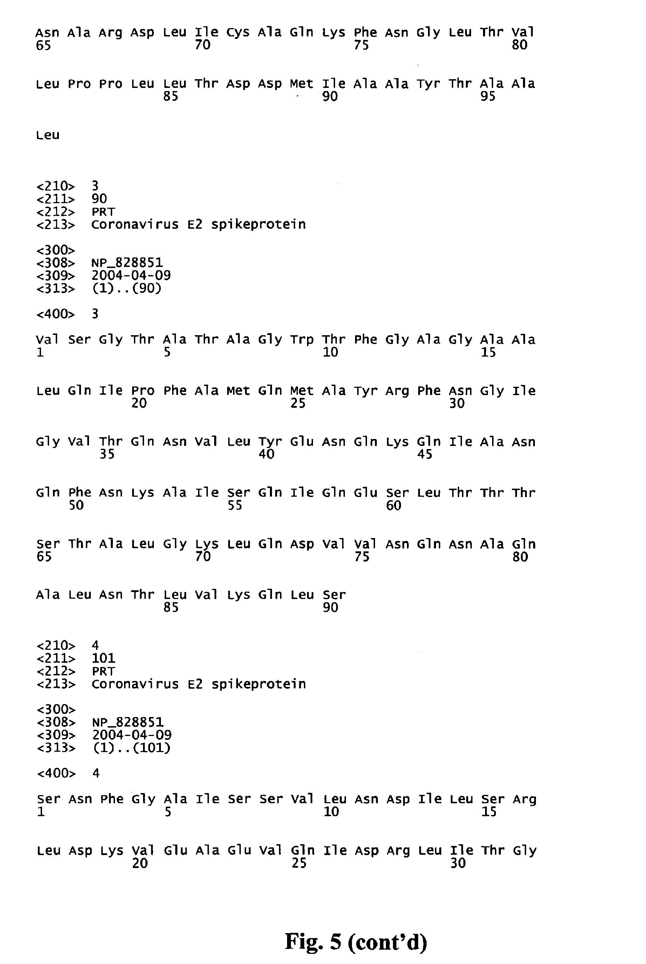

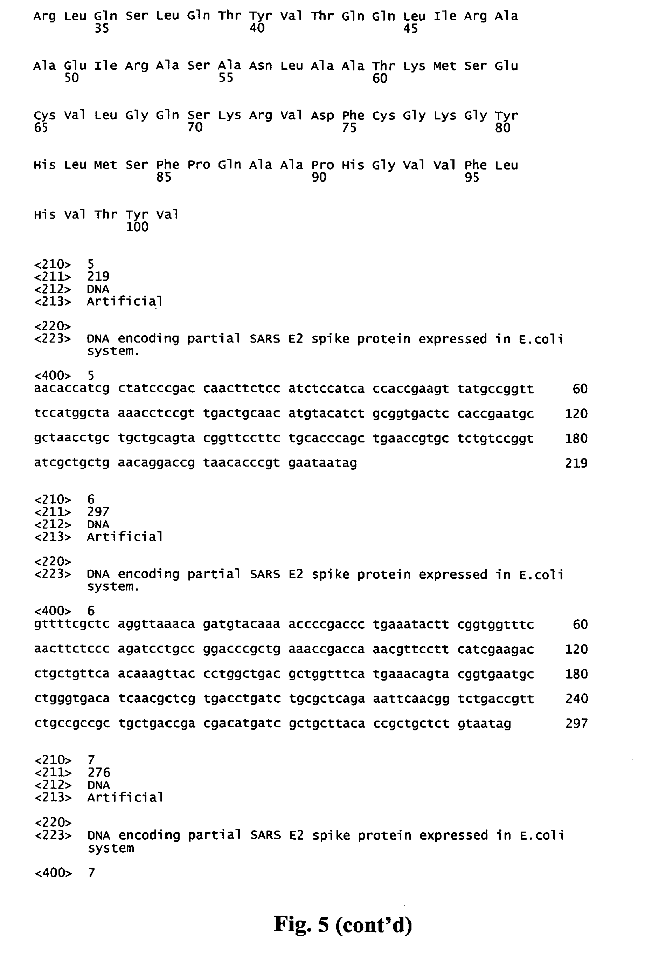

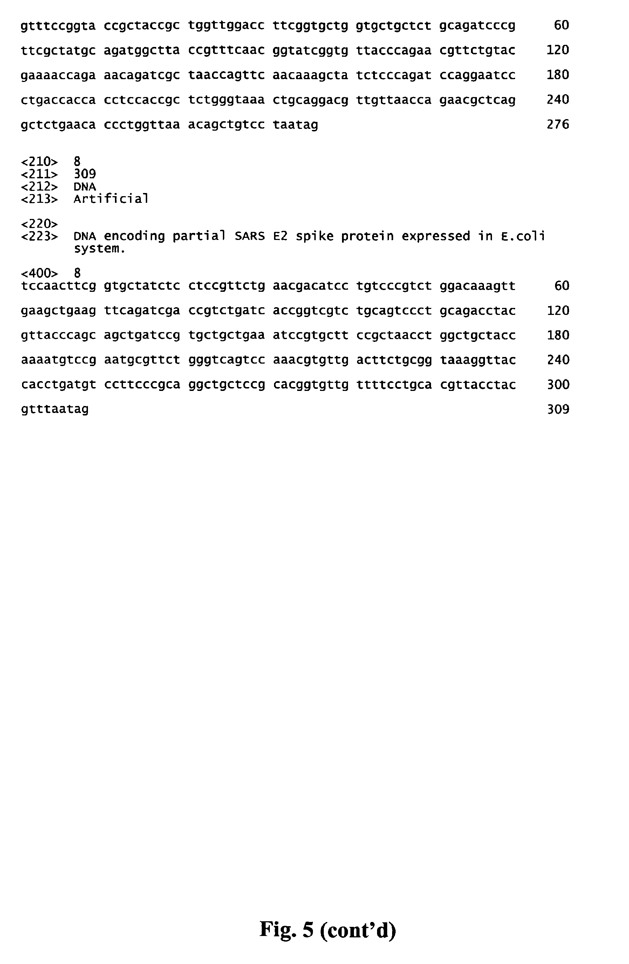

To achieve the object, the present invention discloses a super-antigen fusion protein, comprising a peptide fragment for the same gene encoding at least a partial SARS E2 spike protein. Moreover, a translocating peptide fragment is disclosed for cell binding and translocation, wherein the peptide fragment encoding SARS E2 spike protein includes SEQ ID NO.1, SEQ ID NO.2, SEQ ID NO.3, or SEQ ID NO.4 (see Figure 5).

The present invention also comprises a nucleic acid sequence encoding super-antigen fusion protein, the sequence including SEQ ID NO.5, SEQ ID NO.6, SEQ ID NO.7, or SEQ ID NO.8 (see Figure 5); wherein, the specific proteins are expressed by the nucleic acid in an *E. Coli* expression system.

The present invention further comprises a peptide for T lymphocyte binding, and the peptide contains at least a partial SARS E2 spike protein, including SEQ ID NO.1, SEQ ID NO.2, SEQ ID NO.3, or SEQ ID NO.4.

The present invention also comprises a pharmaceutical composition for a peptide binding to a T lymphocyte, comprising a peptide fragment encoding at least a partial SARS E2 spike protein, wherein the peptide fragment encoding SARS E2 spike protein includes SEQ ID NO.1, SEQ ID NO.2, SEQ ID NO.3, or SEQ ID NO.4.

The peptide sequence of the present invention further comprises a nucleic acid or translocating peptide. The sequence of the translocating peptide is not limited in its category, and mainly has the ability to transport linking peptides into a cell. Preferably, the first and second functional domains of *Pseudomonas* exotoxin A are used for peptide binding and translocating (see US 5,705,163).

The pharmaceutical composition of the present invention also comprises a peptide with amino acids of SEQ ID NO.1, SEQ ID NO.2, or the mixture thereof, and which is used as a vaccine for stimulating a passive immunological response in a subject. A peptide with amino acids of SEQ ID NO.3, SEQ ID NO.4, or the mixture thereof is used for producing a vaccine for the SARS virus in a subject.

The super-antigen fusion protein of the present invention comprises four peptide fragments with their amino acid sequences consensus to SARS E2 spike protein sequence (NP_828851) from National Center for Biotechnology Information (NCBI) database. In order to synthesize the SARS E2 spike protein *ex vivo,* the present invention employs the method disclosed in TW 92126644, to modify the nucleic acid without altering the encoded amino acid for the SARS E2 spike protein partial sequence synthesizing. The modified nucleic acid includes SEQ ID NO.5, SEQ ID NO.6, SEQ ID NO.7, or SEQ ID NO.8.

Other objects, advantages, and novel features of the invention will become more apparent from the following detailed description when taken in conjunction with the accompanying drawings.

### BRIEF DESCRIPTION OF THE DRAWINGS

FIG. 1 shows the plasmid map of the embodiment 1 in the present invention. 1A is pET-PE-SA; 1B is pET-PE-SB; 1C is pET-PE-SC; and 1D is pET-PE-SD.

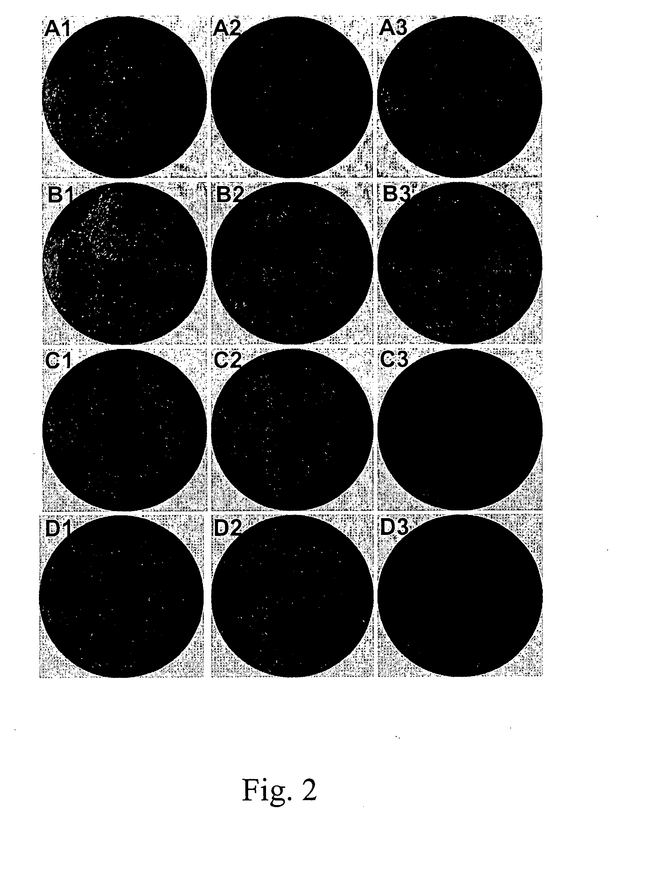

FIG. 2 is the enzyme-linked immunospot assay (ELISPOT assay) of the embodiment 3B in the present invention. The result shows the interactions of the fusion proteins (PE-SA, PE-SB, PE-SC and PE-SD) and the monocytes of human peripheral blood after various incubation periods. A1 is the result with PE-SA fusion protein; A2 is the result with PE-SA fusion protein after 7 days incubation; A3 is the result with PE-SA after 14 days incubation. B 1 is the result with PE-SB fusion protein; B2 is the result with PE-SB after 7 days incubation; B3 is the result with PE-SB after 14 days incubation. C1 is the result with PE-SC fusion protein; C2 is the result with PE-SC after 7 days incubation. C3 is the result with PE-SC after 14 days incubation. D1 is the result with PE-SD fusion protein; D2 is the result with PE-SD after 7 days incubation; D3 is the result with PE-SD after 14 days incubation.
FIG 4 is the antibody titer determination of the Embodiment 4 in the present invention. A, B, C, and D represents the fusion proteins consisting of the PE translocation peptide and SA, SB, SC, or SD peptide fragments respectively. The solid bar represents the present fusion protein, and the hollow bar represents the peptide simply encoding the PE translocation protein.
FIG. 5 illustrates the sequence of SEQ ID NO.1, SEQ ID NO.2, SEQ ID NO.3, SEQ ID NO.4, SEQ ID NO.5, SEQ ID NO. 6, SEQ ID NO. 7 and SEQ ID NO. 8.

### DETAILED DESCRIPTION OF THE PREFERRED EMBODIMENT

### Embodiment 1 Synthesis of Peptide Encoding a Partial SARS E2 Spike Protein

SARS E2 spike protein sequence (accession number: NP_828851, SEQ ID No.9) is found in the National Center for Biotechnology Information (NCBI). Position 680 to 1050 of the amino acid sequence is divided into four fragments for nucleic acid modification, and fragment synthesis and amplification, resulting target amino acid sequences *ex vivo.*

The present invention employs a method disclosed in TW 92126644, and expresses heterogonous virus spike protein in an *E. Coli* expression system. The importance of the modification is to alter a single nucleotide without effecting the original amino acid expression, and further to express specific proteins well in an *E. Coli* host system.

The commercial *E. Coli* plasmid pET230 is used as a template for amplifying nucleic acid fragments with polymerase chain reaction (PCR). In the present embodiment, four synthetic nucleotide fragments are SA, SB, SC and SD peptides. There are 29 pairs of primers used in the fragment amplification, and all the primer sequences are listed in Table 1. The forward primers (F1) are homologous to *E. Coli* plasmid pET230 partial sequence.

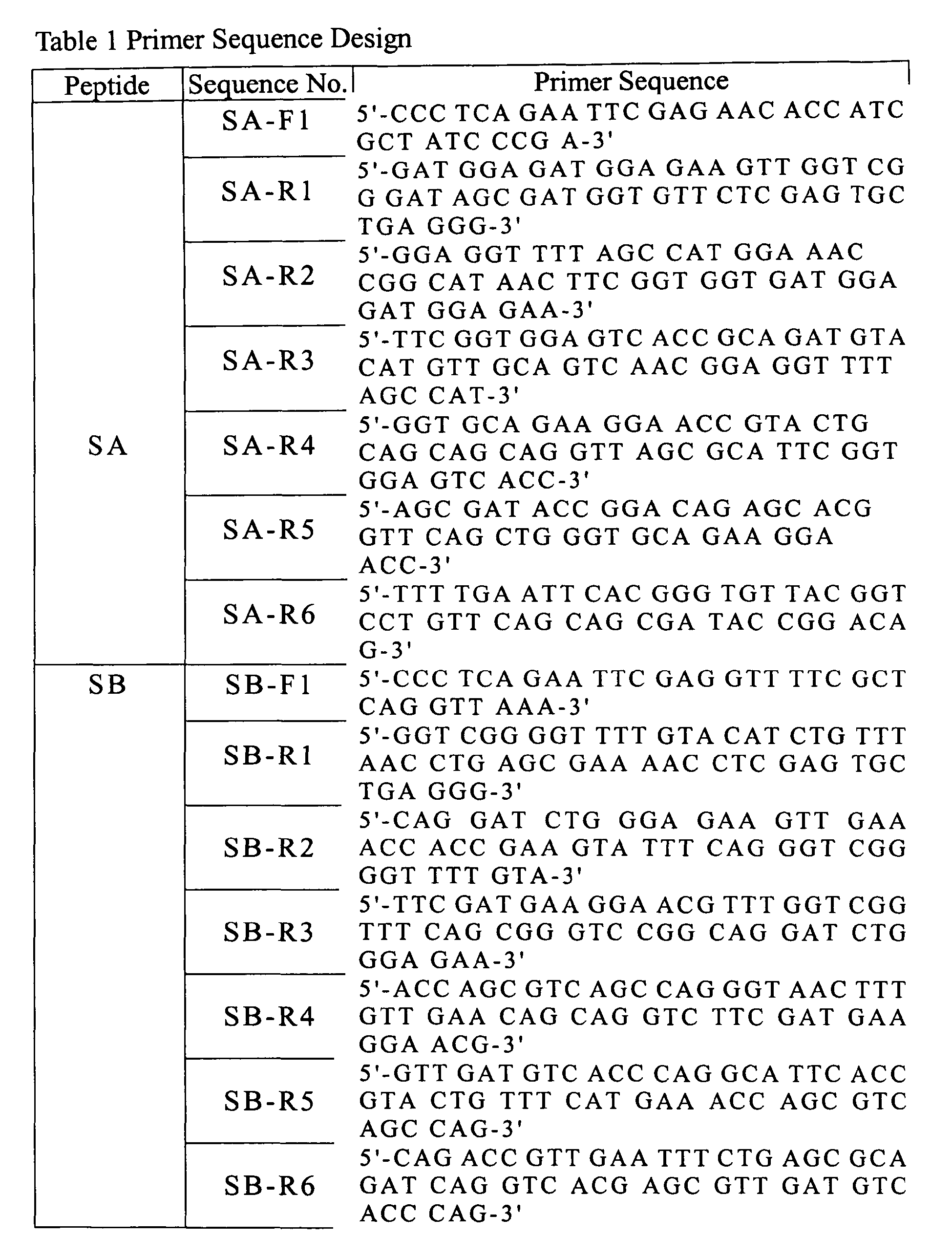

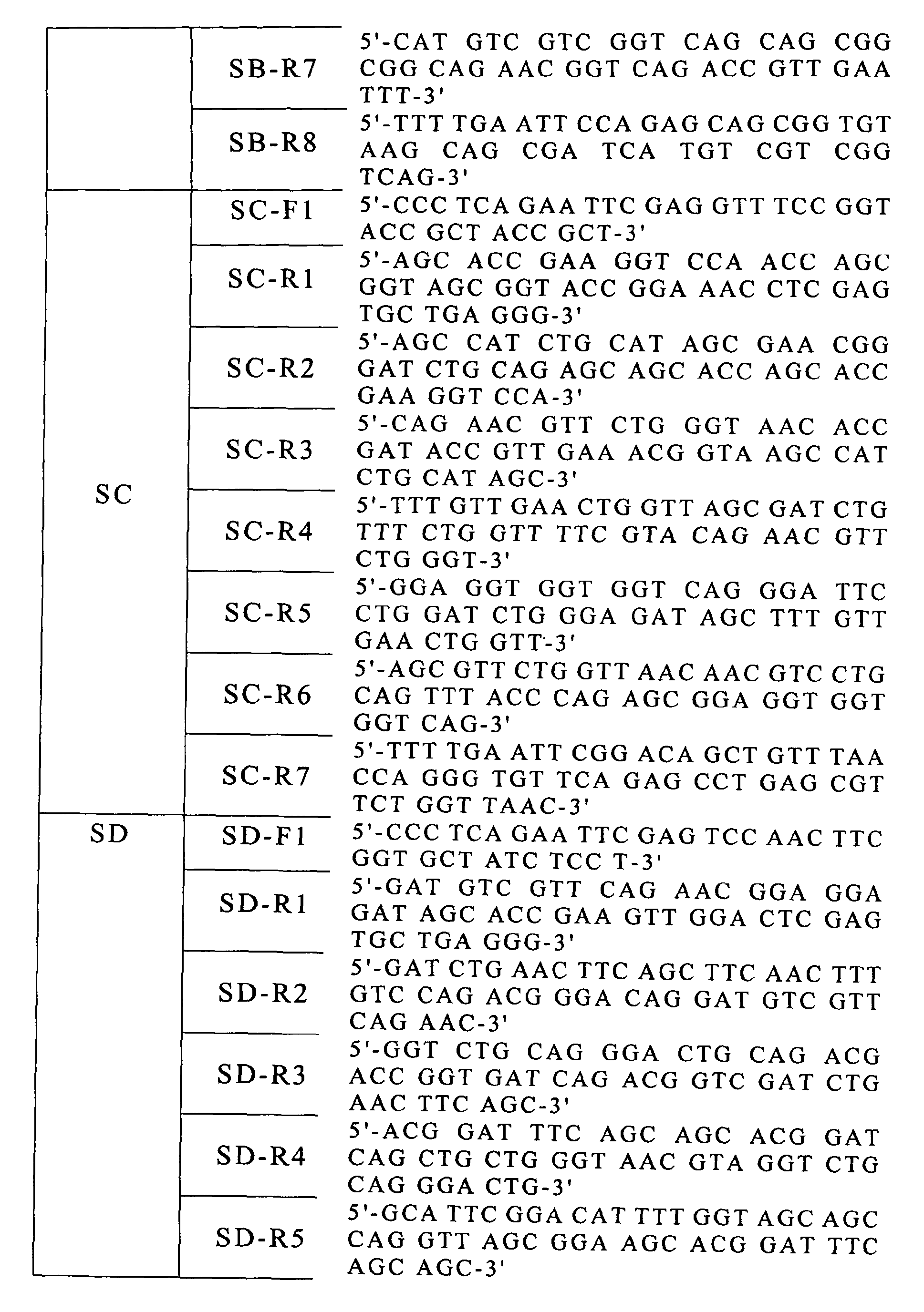

These four peptides are amplified for several times (SA for 6 times, SB for 8 times, SC for 7 times, and SD for 8 times) from PCR by coupling the same forwarded primers (SA-F1, SB-F1, SC-F1, and SD-F1) with various reversed primers (SA-R1~R6, SB-R1~R8, SC-R1~R7, and SD-R1~R8). The amplification conditions are: a first cycle for 5 min at 95 °C; a second cycle for 1 min at 94°C, 0.5 min at 55°C, and 1 min at 72°C of 20 cycles; and a third cycle for 1 min at 72°C .

After amplification, the PCR products are visualized in the agarose gel electrophoresis. The representatives are cut and eluted from gel slices.

### Embodiment 2 Plasmid Construction

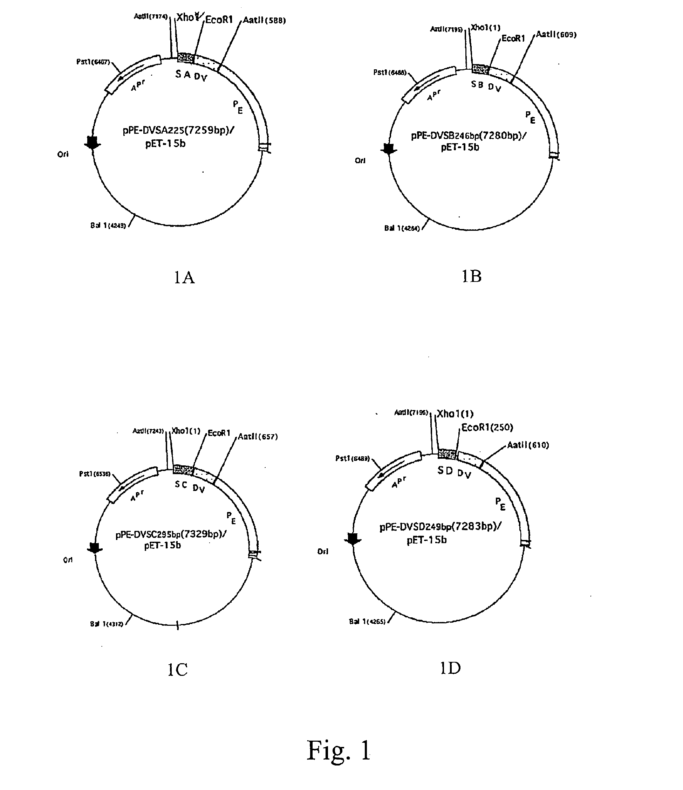

Four nucleotides SA, SB, SC, and SD from PCR, and plasmid pET-PE which contains binding domain and translocating domain of *Pseudomonas* exotoxin A (see Liao C.W. et al., Applied Microbiol Biotechnol 143:498-507, 1995), are ligated together in the restriction enzyme site respectively. Four constructed fusion plasmids are obtained and named pET-PE-SA, pET-PE-SB, pET-PE-SC, and pET-PE-SD (Figure 1).

### Embodiment 3 Protein Expression and Purification

According to the method from Sambrook (Sambrook *et al.,* 1989), the four plasmids pET-PE-SA, pET-PE-SB, pET-PE-SC, and pET-PE-SD are expressed into proteins by using IPTG induction methods.

The *E. Coli* strain for the protein (peptide) expression is BL21 (DE3) pLysS. First, the picked colony is inoculated into 100 ml of LB medium containing 200 µg/ml of ampicillin until OD₅₅₀ reaches approximate 0.3. Second, 1 mM IPTG (isopropylthio-β-D-galactoside, Promege, USA) is added into medium, the incubation is carried on for 90 min, and the cell is centrifuged and collected. The membrane of cells carrying the target protein are loosen by freeze-thaw repeatedly, and then 10 ml lysis buffer (which contains 0.9 mg/ml lysozyme, 1ml PMSF and 0.064 mg/ml DNaseI) is added for 10 min treatment at room temperature. Then, 1ml 10% Triton X-100 is added for another 10 min treatment at room temperature. The mixture is centrifuged in 12000xg for 10 min to collect the protein. Then, the collected inclusion body is dissolved in 4 ml 8M urea..

Furthermore, the commercial pET His-Taq purification system (Novagen, USA) is performed according to the instructions. The cell inclusion body is dissolved in 4 ml ice cold binding buffer (which contains 5 mM imidazole, 0.5 M NaCl, and 20 mM Tris-HCl, pH7.9) with sonication until the inclusion body disperses. Then, the mixture is centrifuged in 1200xg for 15 min at 4°C , and the supernatant is poured into a column (which is the His-Bind metal chelatiing resin immobilized with Ni²⁺). Finally, the protein binding to column is eluted and collected with a buffer (which contains 0.5 M imidazole, 0.5 M NaCl, and 20 mM Tris-HCl, pH7.9).

Four fusion proteins, i.e. PE-SA, PE-SB, PE-SC, and PE-SD, have been expressed from fusion plasmids in the same process.

### Embodiment 4 Cellular Immunity Test

### A. Isolation of peripheral blood monocytes

The blood of a healthy adult is obtained and mixed in the Hanks' balanced salt solution (Hanks' balanced salt solution kit, Life Technologies, Rockville, MD) at a 1:1 ratio.

According to the method from Sacerdote (Sacerdote, 1991#1979), the Ficoll-Paque solution (Amersham Biosciences, Uppsala, Sweden) is used for isolating peripheral blood monocytes. The mixture of blood and Ficoll-Paque solution is centrifuged (600xg, 30 min), and then the solution with monocytes is removed to another centrifuge tube. The mixture is washed twice with Hanks' balanced salt solution and centrifuged in 1200xg for 10 min to obtain the isolated peripheral blood monocytes.

According to the method from Gong (Gong, 2000# 1976), the immature peripheral blood monocytes have the potential to proliferate into dendritic cells (DCs). First, the peripheral blood monocytes are dispersed in the cell culture medium to allow the cells to attach to the culture dish, whereafter the cells incubate for 2 hr at 37°C . Then, the unattached cells are removed. The cells are incubated with 1% human serum RPMI 1640 (Mediatech, Herndon, VA) medium and GM-CSF (800 IU/ml) and IL-4 (500 U/ml) for 6 days. The cytokines are added on Day 0, Day 2, or Day 4. On Day 6, the unattached cells are collected to analyze the molecular marker of the dendritic cell.

The collected dendritic cells are washed with a FACScan buffer consisting of PBS, 0.2% FBS, and 0.5% sodium azide. Further, the FITC- or PE-conjugated anti-human antibodies (CD1a, CD3, CD56, CD80, HLA-A, B, C, and HLA-DR; PharMingen, San Diego, CA) are added to the cells for 30 min on ice. Again, the cells are washed with the FACScan buffer. The data is analyzed with FACScalibur flow cytometer (Becton Dickinson, Mountain View, CA) and "CellQuest" software.

### B. IFN-γ enzyme-linked immunospot assay (ELISPOT assay)

According to the articles from Miyahira *et al.* (Miyahira, 1995#677) and Murali-Krishna *et al.* (Murali-Krishna, 1998 #698), enzyme-linked immunospot assay is modified to estimate the specificity for SA, SB, SC, and SD antigens with CD8+ T lymphocyte.

A layer of anti-human INF-γ antibody (10 µg/ml; PharMingen) in PBS (50 µl) is covered on a 96-well dish (Millipore, Bedford, MA) and cultured at 4°C overnight. Then, the cultured wells are washed and coated with 10% fetal bovine serum (FBS) medium. Moreover, different amounts of fresh or cultured peripheral blood monocytes are prepared, and further mixed with proliferated dendritic cells at a 10:1 ratio (as described in the Embodiment 3). An amount of 1 × 10⁶ of dendritic cells are added into each cultured well coated with FBS, wherein the cells are untreated or treated with four fusion proteins respectively, i.e. PE-SA, PE-SB, PE-SC, and PE-SD, for 2 hr.

After incubation, the cultured wells are washed again. A biotin-conjugated rat anti-human INF-γ antibody (5 µg/ml; PharMingen) in PBS (50 µl) is added and incubated overnight at 4°C . Then, the dish is washed for six times. 1.2 µg/ml of avidin-alkaline phosphatase (Sigma, St. Louis, MO) in 50 µl PBS is added and incubated for 2 hr at room temperature. Further, 50 µl of 5-bromo-4-chloro-3-indolyl phosphate/nitroblue tetrazolium solution (Boehringer Mannheim, Indianapolis, IN, USA) is added for 20 min at room temperature, and the spots are observed. The substrate solution is removed, and the cultured wells are washed with water to stop the reaction. Finally, the cultured wells are dried and the spot amount is counted under an anatomic microscopy.

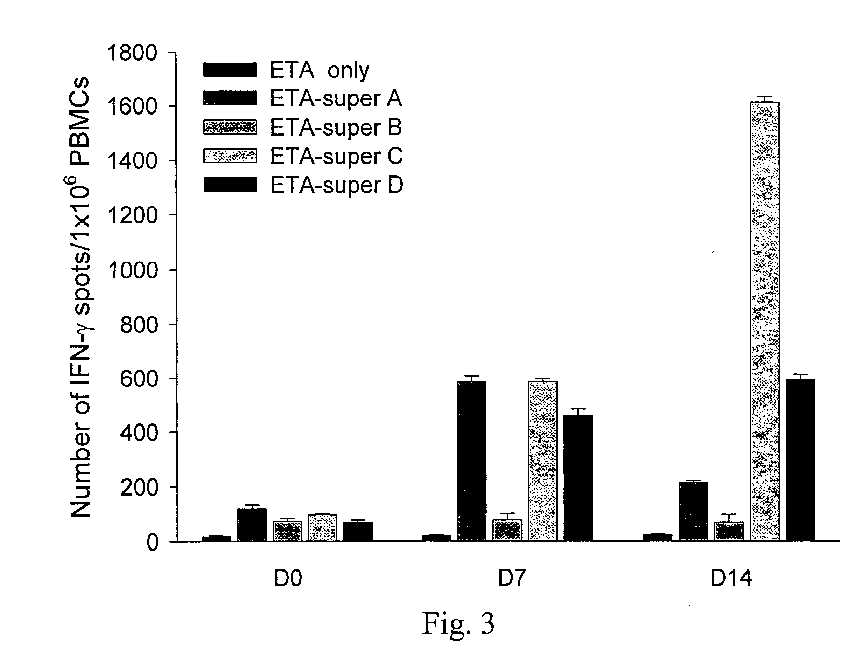

An enzyme-linked immunospot assay is used for evaluating the amount of CD8+ T lymphocytes reflected from PE-SA, PE-SB, PE-SC, and PE-SD fusion protein treatment. Figure 2 is the result of enzyme-linked immunospot assay, which represents the reactions from different incubation times. The spots are counted and shown in a bar chart as in Figure 3. From the figure, it is observed that on the seventh day after immunizing, serum IFN-γ production is elevated in mice injected with PE-SA, PE-SC, and PE-SD fusion protein. On the fourteenth day after immunizing, PE-SC fusion protein will exhibit a severe immunological response and produce a high amount of IFN-γ in mice.

### Embodiment 5 Animal Immunity Test

### A. Animal preparation

Six to eight week-old female mice C57BL/6J are purchased from National Taiwan University (Taipei, Taiwan), and are fed in the Animal Center of National Taiwan University Hospital.

### B. Animal immunity

The fusion proteins, i.e. PE-SA, PE-SB, PE-SC, and PE-SD, prepared from Embodiment 2 are injected individually into each mice in an amount of 100 µg for immunological response observation. Meanwhile, another group of mice is prepared for injecting with *Pseudomonas* exotoxin A peptide only as the control. The administering schedule is Day 0, Day 14, and Day 28 while initiating the experiment.

### C. Antibody specificity test

According to the method from Cheng (Cheng, 2001 #1486), i.e. enzyme-linked immunoabsorbent assay (ELISA), serum PE-SA, PE-SB, PE-SC and PE-SD antibody specificity is evaluated. A 96-well dish is covered with a layer of PE-SA, PE-SB, PE-SC, and PE-SD (5 µg) fusion proteins for overnight culturing at 4°C . Then, 20% fetal bovine serum (FBS) in PBS is coated into culture well. Mice serum is obtained after 14 days of immunization, and serially diluted with PBS and then added into cultured wells for 2 hr at 37°C. Furthermore, cultured wells are washed with 0.05% Tween 20 in PBS, and a 1:2000 peroxidase-conjugated rabbit anti-mouse IgG antibody (Zymed, San Francisco, CA) is added for 1 hr at room temperature. After washing, 1-Step Turbo TMB-ELISA (Pierce, Rockford, IL) is added to exhibit the color. Finally, the reaction is stopped by adding 1MH₂SO₄. The result is read in the 450 nm absorbance spectra in an ELISA reader.

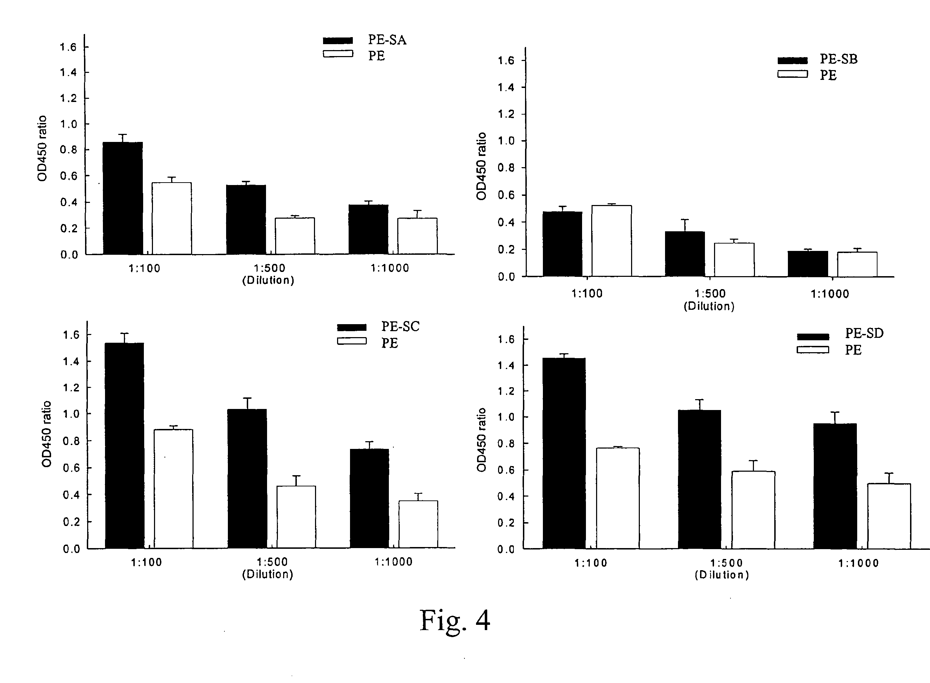

Figure 4 is the titer estimation of specific antibody with the serum diluted serially at the ratio of 1:100, 1:500 and 1:1000. The result shows that the CD8+ cell amount in mice serum injected with PE-SC and PE-SD fusion protein is higher than that of the serum from PE-SA and PE-SB fusion protein injected mice (*P*<0.01, one-way ANOVA). Herein, it is found that PE-SC and PE-SD fusion protein could stimulate higher antibody in animals. Thus, in clinical tests, a subject accepting PE-SC and PE-SD fusion protein will produce an antibody for the SARS virus and protect oneself form infection.

The fusion proteins of the present invention successfully induce antibody production in animals. For large amounts of IFN-γ production induced by PE-SC and PE-SD fusion protein, it is observed that in the present embodiment, the amino sequences of SC and SD peptides are possibly located in the super-antigen positions of SARS virus. Therefore, the excessive immunological response occurs in an antibody-inducting animal injected with PE-SC and PE-SD fusion protein, and the immunological response is not harmful to an animal. For another aspect, the large amount of specific antibodies in animals, induced from PE-SC and PE-SD fusion protein enables the vaccine manufacture in the future and has the potential for SARS therapy.

Furthermore, the present PE-SA and PE-SB fusion protein do not induce excessive immunological response in animals. Thus, it is possible to stimulate passive immunity in an animal tending to be infected with the SARS virus. In animals, the binding mechanism of the SARS spike protein and T lymphocyte surface receptor CD 13 can be blocked by simply administering PE-SA and PE-SB fusion proteins, and thus prevent the SARS virus infection.

Although the present invention has been explained in relation to its preferred embodiment, it is to be understood that many other possible modifications and variations can be made without departing from the spirit and scope of the invention as hereinafter claimed.

## Claims

1. A super-antigen fusion protein comprising:
(a) a peptide fragment encoding at least a partial severe acute respiratory syndrome (SARS) E2 spike protein; and
(b) a translocating peptide;
wherein the amino acid of said peptide fragment encoding SARS E2 spike protein includes SEQ ID NO.1, SEQ ID NO.2, SEQ ID NO.3, or SEQ ID NO.4.

2. The fusion protein as claimed in claim 1, wherein said translocating peptide is obtained from *Pseudomonas* exotoxin.

3. A nucleic acid sequence, which expresses specific SARS E2 spike protein in an *E. Coli* expression system, comprising the following sequence:
SEQ ID NO.5;
SEQ ID NO.6;
SEQ ID NO.7; or
SEQ ID NO.8.

4. The nucleic acid sequence as claimed in claim 3, wherein said nucleic acid sequences further comprise a translocating peptide.

5. The nucleic acid sequence as claimed in claim 4, wherein said translocating peptide is obtained from *Pseudomonas* exotoxin.

6. A peptide for binding to T lymphocyte, comprising a peptide fragment encoding at least partial SARS E2 spike protein, and the amino acid sequence of said peptide is:
SEQ ID NO.1;
SEQ ID NO.2;
SEQ ID NO.3; or
SEQ ID NO.4.

7. The peptide as claimed in claim 6, wherein said peptide fragment encoding at least a partial SARS E2 spike protein is expressed in an *E. Coli* expression system.

8. The peptide as claimed in claim 6, further including a translocating peptide.

9. The peptide as claimed in claim 8, wherein said translocating peptide is obtained from *Pseudomonas* exotoxin.

10. A pharmaceutical composition for a peptide binding to a T lymphocyte, comprising a peptide fragment encoding at least a partial SARS E2 spike protein;
wherein the amino acid sequence of said peptide fragment encoding SARS E2 spike protein includes SEQ ID NO.1, SEQ ID NO.2, SEQ ID NO.3, or SEQ ID NO.4.

11. The pharmaceutical composition as claimed in claim 10, further comprising a translocating peptide.

12. The pharmaceutical composition as claimed in claim 11,
wherein said translocating pepetide is obtained from *Pseudomonas* exotoxin A.

13. The pharmaceutical composition as claimed in claim 10, which is used for treating the SARS virus infection.

14. The pharmaceutical composition as claimed in claim 10, further comprising a pharmaceutically acceptable carrier.

15. The pharmaceutical composition as claimed in claim 10,
wherein said peptide including SEQ ID NO.1, SEQ ID NO.2, or the mixture thereof is used as a vaccine for stimulating passive immuniyt.

16. The pharmaceutical composition as claimed in claim 10,
wherein said peptide including SEQ ID NO.3, SEQ ID NO.4, or the mixture thereof is used for producing a vaccine for the SARS virus.
